(19)

**Europäisches Patentamt**

**European Patent Office**

**Office européen des brevets**

(11)  **EP 4 374 871 A1**

(12) **EUROPEAN PATENT APPLICATION**
published in accordance with Art. 153(4) EPC

(43) Date of publication:
**29.05.2024  Bulletin 2024/22**

(21) Application number: **22845498.9**

(22) Date of filing: **22.07.2022**

(51) International Patent Classification (IPC):
**A61K 38/44** (2006.01)      **A61K 31/728** (2006.01)
**A61K 31/198** (2006.01)      **A61P 27/02** (2006.01)

(86) International application number:
**PCT/ES2022/070486**

(87) International publication number:
**WO 2023/002088 (26.01.2023 Gazette 2023/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **23.07.2021  ES 202130717**

(71) Applicants:
• **Fundación para el Fomento de la Investigación Sanitaria y Biomédica de la Comunitat Valenciana (FISABIO)**
**46020 Valencia (ES)**
• **Universitat Politècnica de València**
**46022 Valencia (ES)**
• **Consejo Superior de Investigaciones Cientificas. (CSIC)**
**28006 Madrid (ES)**

• **Universidad Cardenal Herrera Ceu San Pablo**
**46115 Alfara del Patriarca, Valencia (ES)**
• **Universitat de València**
**46010 Valencia (ES)**

(72) Inventors:
• **ESCOBAR BEDIA, Francisco Javier**
**46022 Valencia (ES)**
• **MAYORDOMO FEBRER, Aloma Tadea**
**46115 Alfara del Patriarca, Valencia (ES)**
• **PINAZO DURÁN, Maria Dolores**
**46020 Valencia (ES)**
• **SABATER PICOT, María José**
**46022 Valencia (ES)**
• **SANZ GONZÁLEZ, Silvia María**
**46020 Valencia (ES)**

(74) Representative: **Elzaburu S.L.P.**
**Edificio Torre de Cristal**
**Paseo de la Castellana 259 C, planta 28**
**28046 Madrid (ES)**

(54) **COMPOSITION BASED ON THE ANTIOXIDANT ACTIVITY OF THE ENZYME SUPEROXIDE DISMUTASE AND APPLICATION THEREOF IN EYE DISEASES**

(57)    The present invention relates to the composition containing an enzyme belonging to the group of superoxide dismutases (SOD), or any SOD mimic and the like together with hyaluronic acid and EDTA. Said composition is part of a pharmaceutical formulation which promotes or maintains eye health through the inhibition of cell and tissue damage caused by free radicals, so it is useful for the treatment and/or prevention of eye diseases that occur with oxidative stress, or as an adjunct to other existing current treatments, as well as in post-operative treatments to treat and/or prevent inflammatory or oxidative stress adverse reactions.

EP 4 374 871 A1

**Description**

**TECHNICAL FIELD OF THE ART**

The present invention relates to a composition containing an enzyme belonging to the group of superoxide dismutases (SOD), or any SOD mimic, together with hyaluronic acid and EDTA or any of its salts. Said composition is part of a pharmaceutical formulation which promotes or maintains eye health through the inhibition of cell damage caused by free radicals, so it is useful for the treatment or prevention of eye diseases that occur with oxidative stress, or as an adjuvant to other existing current treatments. Likewise, it is also useful for post-operative use of surgery of the anterior segment of the eye, and/or laser treatment in which an inflammatory response and oxidative stress occur.

Therefore, the present invention belongs to the field of pharmacology or pharmaceutical chemistry.

**BACKGROUND OF THE INVENTION**

Ophthalmological practices attend daily to a large number of patients with diseases that potentially cause visual impairment and blindness, and many other consultations for eye problems of a very diverse nature. We must highlight those that affect refractive errors (myopia, hyperopia, astigmatism, presbyopia), the eye surface (dry eye syndrome, keratoconus, corneal dystrophies), and those that affect the retina or the optic nerve (retinopathies, retinal vascular accidents, macular degeneration and/or optical neuropathies), as well as neurodegenerative diseases (glaucoma, hereditary retinal dystrophies and genetic syndromes). All of them, from the mildest to the most serious, have in common pathogenic conditions such as oxidative stress, inflammation and/or apoptosis.

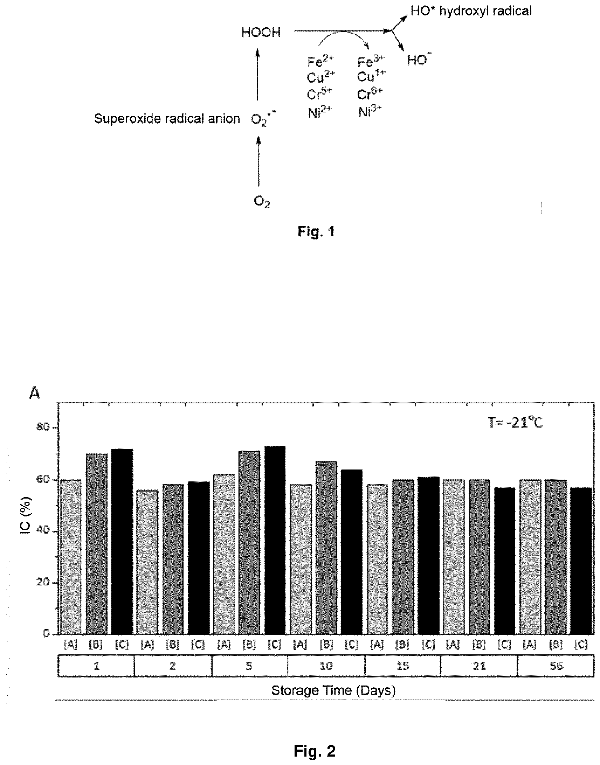

Oxidative stress arises when there is an imbalance between the production of reactive oxygen species (ROS) and the capacity of the biological system to counteract them (antioxidant systems) in favour of the former. In other words, oxidative stress is due to the increase in pro-oxidant activity and/or the reduction of antioxidant defence mechanisms, or to the failure of the repair mechanisms of oxidative damage at the cellular and tissue level. Under normal conditions, oxygen ($O_2$) is tetravalently reduced to $H_2O$. However, the incomplete reduction of oxygen generates ROS, including the superoxide anion ($O_2^-$), which in the presence of metals (Fe, Cu, Cr, Ni) induces the formation of hydrogen peroxide ($H_2O_2$) and hydroxyl radical ($OH^-$), these oxygen species being very harmful to cells, tissues, organs and systems (Figure 1).

In this context, the superoxide dismutase SOD enzyme is the first defence barrier against oxidative aggression, since it exerts a protective role against oxidative stress by catalysing the dismutation of the highly toxic radical called superoxide anion ($O_2^-$) into $O_2$ and $H_2O_2$, which is finally transformed into a totally harmless compound such as water. Since it has been shown that the $O_2^-$ radical is an important mediator of tissue damage and organic dysfunction in different processes (i.e. inflammation, apoptosis, cytotoxicity/excitotoxicity, angiogenesis), and since the SOD enzyme is a powerful antioxidant capable of blocking the formation of $O_2^-$ radical, it has been sought to develop a therapy to normalize the activity of the cellular detoxification system and decrease the levels of EROs at the ocular level, based on the redox activity of the SOD enzyme (S. Di Meo, T. Reed, P. Venditti, V. M. Victor, Role of ROS and RNS Sources in Physiological and Pathological Conditions, Oxid. Med. Cell Longer, 2016, 2016: 1245049).

The use of the SOD enzyme for the treatment of diseases associated with oxidative stress is known in the state of the art. In fact, old inventions describe the use of SOD enzyme-containing pharmaceutical compositions for topical administration in order to prevent and treat eye diseases (US2003/0228299). Furthermore, other inventions such as the one described in WO03/082081 describe the use of a compound (i.e. SOD) in topical, intravitreal, and systemic administration to reduce the amount of EROs metabolites and thus treat intraocular damage.

On the other hand, the administration of SOD orally has been limited due to the degradation of the enzyme through the digestive tract. The route of administration is known to be a critical factor in any therapeutic intervention. To overcome this problem, the use of wheat gliadin (a biopolymer or protein found in wheat flour) to protect the SOD enzyme from digestive degradation (GliSODinR (Isocell)) has been described. In fact, it has been shown that the catalytic activity of SOD is preserved by this biopolymer during oral administration, allowing its administration through this route (I. Vouldoukis, D. Lacan, C. Kamate, P. Coste, A. Calenda, D. Mazier, M. Conti, B. Dugas, Journal of Ethnopharmacology, 2004, 94(1), 67-75).

Parallel to the enzymatic protection against oxidative stress, there are also non-enzymatic products, such as exogenous ones that are taken with food, including vitamins (A, C, E), metals such as Zn, Se, Mn, carotenoids (lutein, zeaxanthin), flavonoids (catechins, quercetins, anthocyanidins), and omega-3 fatty acids, or the endogenous among which glutathione and coenzyme Q10 stand out. All of them are also often used as antioxidants. US2005/0032914, US2010/0159029 and WO2009/129859 documents describe compositions for oral administration of lutein, zeaxanthin in combination with other antioxidant products which protect vision, decrease the symptoms of various eye diseases and slow down the course of dry or atrophic macular degeneration, and promote general eye health.

On the other hand, CN101243875 describes a preparation containing lutein and beta-carotene to increase the activity of SOD protected by gliadin. Apparently lutein alone is less efficient in increasing the activity of the SOD enzyme than in combination with b-carotene, highlighting this synergistic effect. Another more recent invention describes the preparation of a preparation to be administered orally containing a plant extract of the SOD enzyme protected with a polymer in association with lutein and zeaxanthin (US 2013/0195985).

## EXPLANATION OF THE INVENTION

The inventors have developed a stable isotonic solution that incorporates SOD, hyaluronic acid and EDTA or their salts that distinguishes this preparation from others that are currently marketed, since, in addition to the moisturizing, lubricating, regenerating and protective properties on the eye surface provided by hyaluronic acid, EDTA salt acts as a chelating agent for free metal cations (i.e. copper, iron, manganese,...) that can catalyse oxidation reactions, so it exerts a synergistic enhancing effect on the antioxidant activity of the SOD enzyme. On the other hand, with the formulation of all these components in the same preparation, a long-term stable solution of the enzyme is achieved (2 months). In addition, the data from the tests carried out with human tears and plasma of experimental animals demonstrate that the composition of the invention is effective and tolerable to regulate the total antioxidant activity and the inflammatory activity dependent on interleukin 6, so that they can be applied to eye surface diseases in which both harmful mechanisms are involved, specifically in the ocular surface dysfunction syndrome - dry eye.

In a first aspect, the present invention refers to a composition comprising:

- superoxide dismutase (SOD) enzyme, in a concentration between 0.002 U/mL and 30,000 U/mL.
- hyaluronic acid or any of its salts, in a concentration between 0.0001 and 10% weight/volume with respect to the total composition,
- ethylenediaminetetraacetic acid (EDTA) or any of its salts, in a concentration between 0.0001 and 10% weight/volume with respect to the total composition, and
- a pH buffer selected from citrate, phosphate, ascorbate, borate.

The SOD enzyme refers to any of the 4 known isomorphic proteins of this enzyme, whereby, preferably, the SOD of the composition of the invention is selected from Cu/Zn-SOD, Mn-SOD, Ni-SOD and Fe-SOD. More preferably, said SOD is Cu/Zn-SOD.

The SOD enzyme of the composition of the invention refers to SOD enzyme of natural origin (plant, animal or human origin) or of synthetic origin, preferably of animal origin.

In the case of naturally occurring SOD enzyme, it does not come from any genetically modified organism (GMO). In the most preferred embodiment, it is a SOD (Cu/Zn-SOD) enzyme of animal origin from mammalian cells (bovine erythrocytes).

In the case of SOD enzyme of synthetic origin, it refers to recombinant SOD, both of plant and animal origin. For example, a SOD enzyme sequence of human origin cloned and produced in a prokaryotic system, such as for example a bacterium. Or, a SOD enzyme sequence of human origin cloned and produced in a eukaryotic system, such as for example a fungus or a cell of a mammal (i.e. CHO cells). Or, a SOD enzyme sequence of human origin cloned and produced in a viral system, such as for example a baculovirus.

Also considered for the composition of the invention are any mimic of SOD, which are understood as functional analogues of this low molecular weight enzyme; among them, it is worth mentioning metal complexes that act as pharmacological agents blocking the production of radicals, especially the superoxide radical anion, among others. Also organic, inorganic and metalloorganic molecules that act as functional analogues blocking the formation of radicals.

Preferably, the SOD of the composition of the invention is in a concentration between 0.2 and 20.000 U/mL, preferably between 1 and 500 U/mL, more preferably between 10 and 250 U/mL and still more preferably between 15 and 60 U/mL. In the present invention 1 mg of SOD can be estimated approximately as 4700 U according to the manufacturer Sigma-Aldrich and these units in the present invention are referred to the total mL of the composition described herein.

Preferably, the hyaluronic acid of the composition is in a concentration between 0.05 and 5% weight/volume with respect to the total composition and even more preferably 0.1% weight/volume with respect to the total composition.

Preferably, the EDTA of the composition is in a concentration between 0.05 and 5% weight/volume with respect to the total composition and more preferably 0.1% weight/volume with respect to the total composition.

Preferably, the EDTA of the composition of the invention is in sodium salt form.

Preferably, the above-mentioned possible buffers are in the form of sodium salt. Preferably, the buffer of the composition of the invention is sodium borate.

Preferably, the composition of the invention further comprises a salt selected from NaCl, KCl, $MgCl_2$, $CaCl_2$. More preferably, the salt is NaCl.

Preferably, the composition of the invention is characterized by having a pH between 7 and 7.5. More preferably, characterized by having a pH of 7.3.

Another aspect of the invention relates to a pharmaceutical composition comprising the composition as described above and at least one pharmaceutically acceptable carrier.

Preferably, this pharmaceutical composition is formulated in the form of eye drops, artificial tear solution, solution for injection, spray, gel, ointment or cream.

Another aspect of the invention relates to the composition as described above for use as a medicinal product.

Another aspect of the invention relates to the composition as described above for use in the treatment or prevention of eye diseases.

Preferably, the eye disease is selected among dry eye syndrome, conjunctivitis with inflammatory component, chronic blepharitis, non-infectious keratitis, keratoconus, non-infectious uveitis and/or glaucoma.

Preferably, the composition of the invention may also be used for the treatment and/or prevention of the inflammatory response and oxidative stress produced in post-operative eye surgery, particularly of the anterior segment of the eye and/or laser treatments.

Examples of the administration route of the composition of the invention include systemic administration (for example, oral administration or injection), topical administration (for example, eye instillation, eye ointment, artificial tears), periocular administration (for example, administration of sub-Tenon's capsules), conjunctival administration, intraocular administration, subretinal administration, suprachoroidal administration, retrobulbar administration, and the like. The administration route of the composition of the present invention can be appropriately determined according to whether the application to an eye disease associated with oxidative stress is aimed at prophylaxis or treatment and the like. The preferred route of administration is an ophthalmic topical administration.

The composition of the present invention is preferably administered to a particularly human subject, either after diagnosis of the risk of eye disease, before initiating a possible treatment thereof (i.e. as prophylactic treatment) or after the onset of a diagnosed eye disease (as therapeutic treatment). The treatment plan can be appropriately determined according to the type of active ingredient to be used, dose, route of administration, cause and, when necessary, the level of subjective symptoms of an eye disease.

The dosage (dose) of the composition of the present invention for a subject, particularly a human being, will be an amount sufficient to provide a desired response in the latter, whereby the administration will take place for a reasonable period of time. The dose is appropriately determined according to various factors including the concentration of the active ingredient to be used, the age, the species, the symptoms, the disease state, the body weight and the severity of the disease of the administration subject, the route, the time and the frequency of administration and the like. The dose may also be appropriately controlled according to the route, timing, and frequency of administration and the like. Depending on the symptom or disease state, long-term treatment involving the administration of several doses per day may be necessary.

The composition of the present invention preferably contains a "pharmaceutically acceptable carrier" and, as an active ingredient, SOD in an amount sufficient to prophylactically or therapeutically treat an eye disease such as those mentioned above. The carrier can be any as long as it incorporates the medicinal product and is not particularly limited except when it is limited by physicochemical elements to be considered (for example, solubility and lack of reactivity with the compound or influence on the SOD structure) and route of administration.

The administration form (dosage form) of the composition of the present invention is not particularly limited and can be administered in various forms to achieve a desired therapeutic or prophylactic action.

The composition of the present invention may contain another pharmaceutically active compound as long as it does not inhibit the effect of SOD.

The composition of the present invention may be administered simultaneously with another pharmaceutically active compound as long as the effect of the present invention is not impaired. "Simultaneous administration" means the administration of another pharmaceutically active compound simultaneously (e.g., in the same or a different preparation) by the same or different route of administration before or after administration of the composition of the present invention.

In addition, the present invention provides a method for treating eye diseases comprising a step of administering, to a subject in need of treatment, the composition of the invention in an amount sufficient to treat the subject of the disease and/or prevent the development of possible eye diseases.

## BRIEF DESCRIPTION OF THE FIGURES

Figure 1. Transformation of $O_2$ into EROs.

Figure 2. Graphical representation (in bar chart and dot plot) of the activity (IC%) of the SOD enzyme over time in artificial tear solution at three different concentrations: Composition A: Light grey colour: 20.3 U/mL; Composition B: Dark grey colour 56.4 U/mL; Composition C: Black colour 169.2 U/mL, determined at -21ºC, +4ºC and +25ºC.

**Figure 3.** Representation of weekly body weight for the three study groups. A) Mice treated with SOD 20.3 U/mL, B) Mice treated with SOD 56.4 U/mL and C) Mice treated with SOD 169.2 U/mL.

**Figure 4.** Fluorescein staining test. A) Both eyes before removing excess. B) Right eye and C) Left eye. Both eyes give a negative result, indicative of the absence of corneal ulcers.

**Figure 5.** Intraocular pressures measured weekly in the three groups of mice under study. In the right eyes, tear buffer is instilled as a control (striped bars) and in the left eye, tear buffer + SOD (solid bars) is instilled. The doses of SOD are: A) 20.3 U/mL, B) 56.4 U/mL and C) 169.2 U/mL.

**Figure 6.** Graphical representation of the total antioxidant concentration in the two main groups.

**Figure 7.** The body weight of all animals was measured at baseline on an animal scale and is expressed as the mean + SD in grams.

**Figure 8.** Mouse plasma concentration of IL6 at baseline and at 15 days of treatment with the two concentrations of the formula (SOD1 and SOD2).

**Figure 9.** Concentration in mouse plasma of IL6 at baseline and at 15 days of treatment with the two SOD concentrations (SOD1 and SOD2) tested.

## PREFERRED EMBODIMENT OF THE INVENTION

The present invention is illustrated by the following examples:

Example 1: preparation of ophthalmic compositions.

Composition A: Initially, an isotonic buffered solution of artificial tears is prepared in which the SOD enzyme is incorporated at the concentration of 20.3 U/mL (U/mL = Units/millilitre). The artificial tear solution is prepared in an amber-coloured glass container to protect it from light. The composition of the artificial tear is as follows: hyaluronic acid (0.1%), disodium edetate (0.1%), NaCl and boric acid/sodium borate (pH: 7.3).

Composition B: Initially, an isotonic buffered solution of artificial tears is prepared in which the SOD enzyme is incorporated at the concentration of 56.4 U/mL (U/mL = Units/millilitre). The artificial tear solution is prepared in an amber-coloured glass container to protect it from light. The composition of the artificial tear is as follows: hyaluronic acid (0.1%), disodium edetate (0.1%), NaCl and boric acid/sodium borate (pH: 7,3)

Composition C: Initially, an isotonic buffered solution of artificial tears was prepared in which the SOD enzyme is incorporated at the concentration of 169.2 U/mL (U/mL = Units/millilitre). The artificial tear solution is prepared in an amber-coloured glass container to protect it from light. The composition of the artificial tear is as follows: hyaluronic acid (0.1%), disodium edetate (0.1%), NaCl and boric acid/sodium borate (pH: 7.3).

Example 2: Determination of the antioxidant activity of the compositions of the invention

Determination of SOD enzyme activity in the three prepared compositions A, B and C (20.3 U/mL, 56.4 U/mL and 169.2 U/mL) and at three different temperatures (-20, +4 and +25ºC) is performed periodically by the McCord-Fridovich method. This method indirectly quantifies the ability of the enzyme to catalyse the dismutation reaction of the superoxide anion ($O_2^-$) in $H_2O_2$ and $O_2$ using colorimetric methods. Specifically, it measures the variation of the reduction rate of the nitroblue tetrazolium dye (NBT) by the superoxide anion ($O_2^-$) to formazan ($\lambda$ = 532 nm), based on the variation of the absorbance of the latter. From this absorbance data, the inhibition coefficient IC (%) (eq. 1) of the SOD enzyme in artificial tears at different concentrations and temperatures, this being a crucial data that will provide information on the activity and stability of the enzyme:

$$IC\ (\%) = \frac{R_0 - R_X}{R_0} \cdot 100 \quad (eq.\ 1)$$

*IC (%)= inhibition coefficient expressed as percentage*
*$R_0$ = rate of NBT reduction in the absence of SOD*
*$R_x$ = rate of NBT reduction in the presence of X SOD units*

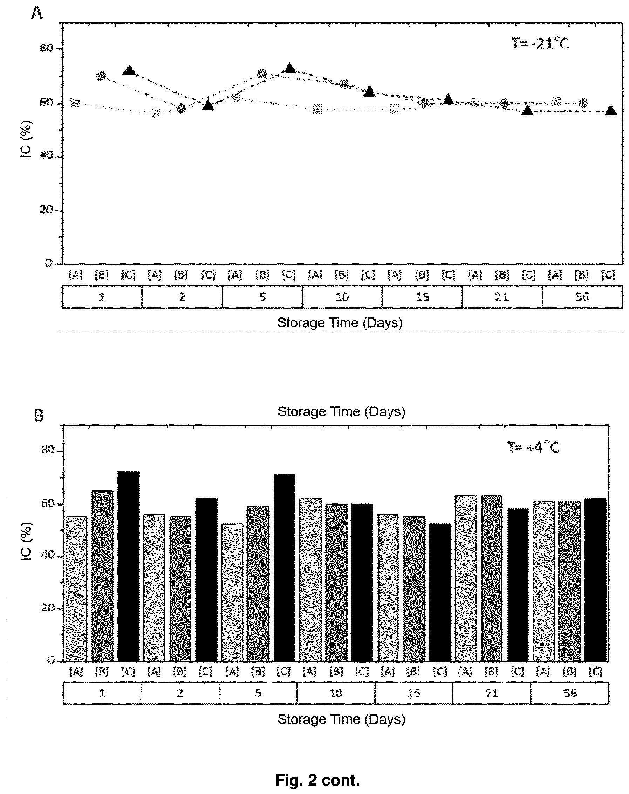

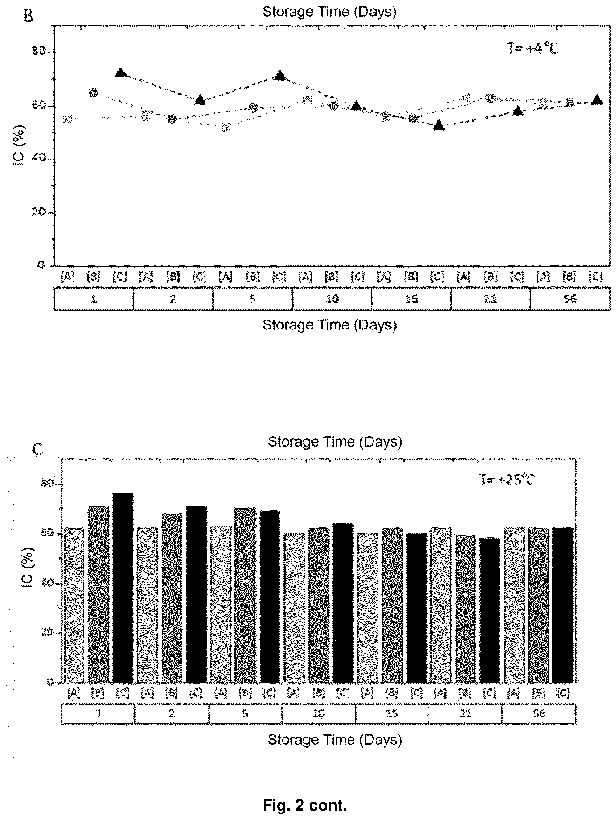

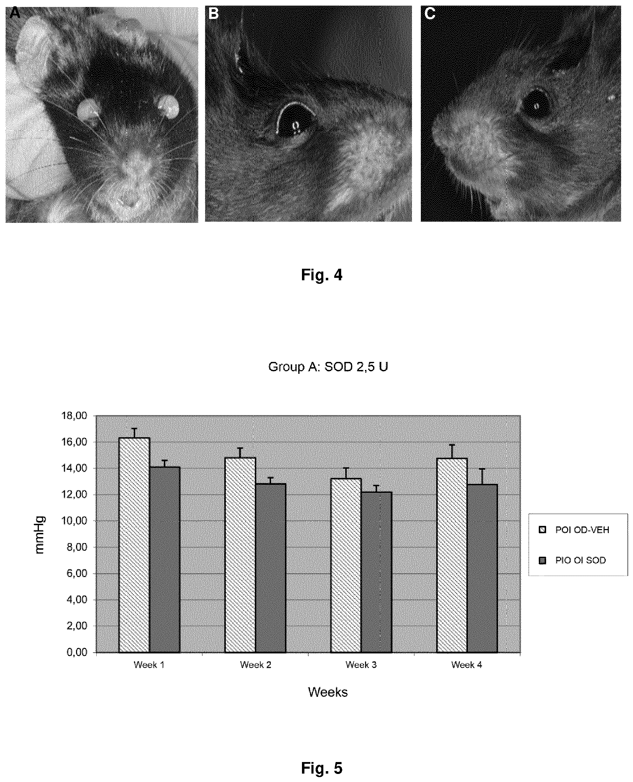

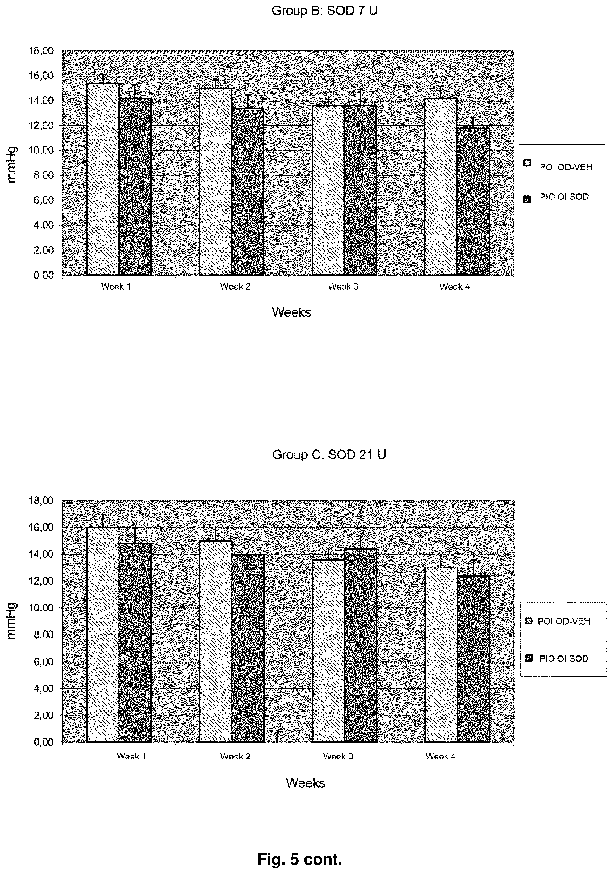

As can be seen from the analytical results represented in Figures 2-5, it can be said that in general the initial IC values (%) of the SOD enzyme remained stable throughout the period of time (2 months) that the tests of the three compositions 1, 2 and 3 (20.3 U/mL, 56.4 U/mL and 169.2 U/mL) lasted, and at different temperatures (-20, +4 and +25ºC).
Therefore, it can be concluded that the SOD enzyme fully maintains its original activity, and even much of it, in isotonic and buffered solutions of artificial tears within the range of concentrations and temperatures studied in the tests and at relatively long storage times (2 months).

Example 3: Biological tests

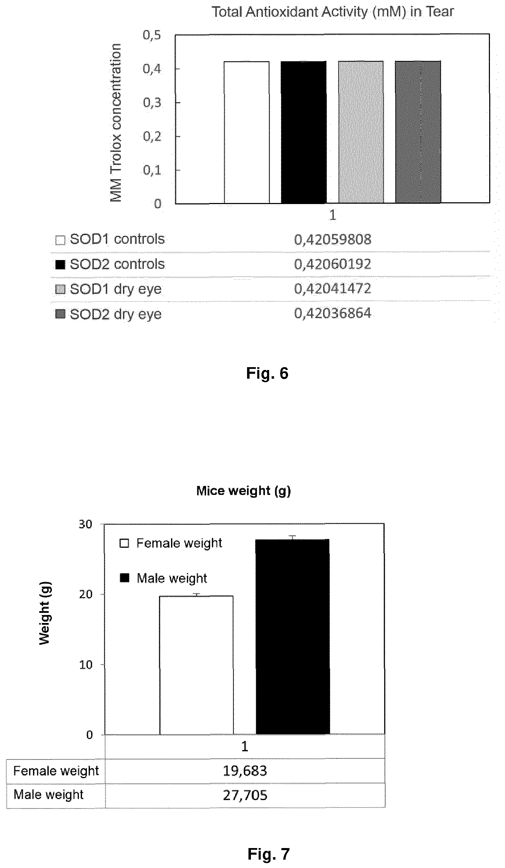

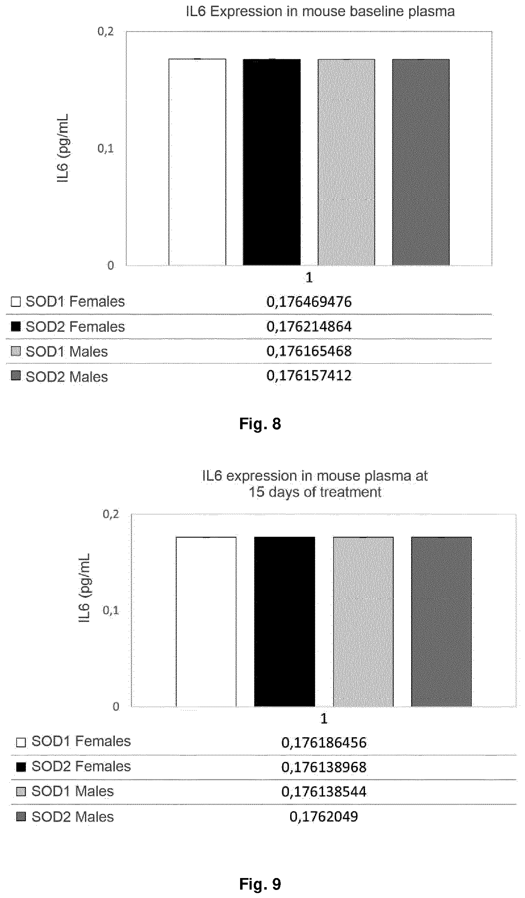

Biological tests were carried out on three groups of mice, each treated with one of the compositions 1, 2 and 3 respectively, for 4 weeks (2 instillations/day; 1 drop/instillation). During the four weeks of the tests, the average body weights of each group of mice did not show statistically significant differences. However, there was a trend to a progressive increase in body weight over time for each group (Figure 6). As for the feeding and behaviour of the animals, no significant differences were found between the different groups of mice.
Continuing with the tests, a continuous and serial eye examination was carried out in the three groups of mice, in which no lesions were observed in the ocular adnexa (orbital region, eyelids and eyelashes) in any of the groups of mice throughout the entire experiment (4 weeks).
Clinical trials showed that all mice presented integrity of the eye surface structures, with good corneal transparency, without the appearance of ulcers or leukomas. For this study, the fluorescein test was used, which showed that the status of the conjunctiva and cornea were totally normal in the three groups of mice during the 4 weeks of the study (Figure 7). Finally, intraocular pressures were measured for each mouse weekly for 4 weeks. The results of intraocular pressures showed no significant differences within each week comparing the three study groups for both the left eye OI (experimental) and the right eye OD (control) (p>0.05) (Figure 8).
Tests in mice indicate that daily instillation (2 instillations per day; 1 drop/instillation) for 4 weeks has no harmful effect on the anterior segment and eye adnexa. This is a very good result that confirms the tolerability of instillation and the absence of toxicity derived from serial administrations.

Example 4: Measurement of the antioxidant activity of the composition of the invention in human tears.

The measurement of the total antioxidant activity of the tear samples is a determination of the composition of all antioxidants present in said sample and provides information on the total antioxidant balance. To measure the antioxidant activity, the "Total Antioxidant Capacity" (TAC) test is used, which is based on the capacity of antioxidants to inhibit the oxidation of 2,2-azino-di-3- [ethylbenzothiazoline] sulphonate (ABTS) to $ABTS^+$. by metamyoglobin. In this case, the amount of $ABTs^+$· produced can be quantified spectrophotometrically by measuring the absorbance of a band at 750 nm (or 405 nm). Initially, since the antioxidants present in the tears can prevent the formation of $ABTS^+$, a drop in the absorbance of this species would give a measure of the TAC, which can be expressed in units of concentration as mM (mmol/L).
Thus, to carry out this trial, tear samples were obtained from 25 volunteers aged between 36 and 85 years old, who attended ophthalmology consultations, which were distributed into two groups: 1) Group of patients with Dry Eye Syndrome (n=15) and 2) Group of healthy controls (n=10). The tears were obtained by the same ophthalmologist on all occasions and with the same extraction technique, to avoid differences derived from the extraction technique. All subjects read the information sheet and signed the informed consent giving consent for participation in the study. The initial interview, the medical history and the ophthalmological examination were used to be randomly selected among those attending the ophthalmological consultations, when it was verified that they met the study inclusion/exclusion criteria. Once extracted, the tears were frozen at -80ºC until processing. Next, the determination of total antioxidant activity (TAC) was carried out using the commercial Antioxidant Assay Kit No 709001, following the protocols and instructions of the manufacturer (Cayman Chemical, Ann Arbor, MI, USA).
The data obtained in the tears extracted from the subjects of the control group are shown in Table 1. As can be seen, similar results were obtained with the two concentrations of SOD tested. The formulation added to the tears of healthy subjects maintained the total antioxidant values within normal ranges.

Table 1. Results obtained from the total antioxidant activity of the tears of the subjects of the control group. They are expressed as mean + SD in millimoles.

| SOD2 Control Group high dose 50.4 U/mL | | | SOD1 Control Group low dose 20.3 U/mL | | |
|---|---|---|---|---|---|
| Medium | 0.42060192 | mM | Medium | 0.42059808 | mM |
| DS | 0.000393131 | mM | DS | 0.00033597 | mM |
| Error | 0.000226975 | mM | Error | 0.00019397 | mM |

These results were similar to those obtained by other investigators in healthy eyes with the same type of test that has been used for these tear samples (Choi W, Lian C, Ying L, et al. Expression of lipid peroxidation markers in the tear film and ocular surface of patients with non-Sjogren syndrome: potential biomarkers for dry eye disease. Curr Eye Res. 2016; 41: 1143- 1149, Seen S, Tong L. Dry eye disease and oxidative stress. Acta Ophthalmologica 2017; 96 (4):e412-e420). In addition, Choi et al., demonstrated with the FRASC technique that the measurement of the total antioxidant of the sample (mean+SD) in tears collected with a capillary tube in 47 healthy individuals from China and the data were similar to the 6 obtained with the CT technique: 0.409 + 0.16 mM (ranging within 0.46-1.02 mM) Choy CK, Benzie IF, Cho P. Ascorbic acid concentration and total antioxidant activity of human tear fluid measured using the FRASC assay. Invest Ophthalmol Vis Sci. 2000;41:3293-8.

Table 2 shows our results in tears obtained from subjects diagnosed with ocular surface dysfunction syndrome - dry eye - distributed in the two subgroups to which the corresponding SOD concentration was added. As can be seen, similar results were obtained with the two concentrations of SOD tested. The formulation added to the tears of patients with eye surface dysfunction - dry eye - was able to reverse and maintain the values of total antioxidant activity within normal.

Table 2. Results obtained from the total antioxidant activity of the tears of the subjects in the group with ocular surface dysfunction syndrome (dry eye). Expressed as mean + SD in millimoles

| SOD2 Dry Eye Group high dose 50.4 U/mL | | | SOD1 Dry Eye Group low dose 20.3 U/mL | | |
|---|---|---|---|---|---|
| Medium | 0.42036864 | mM | Medium | 0.42041472 | mM |
| DS | 0.000690536 | mM | DS | 0.00051986 | mM |
| Error | 0.000398681 | mM | error | 0.00030014 | mM |

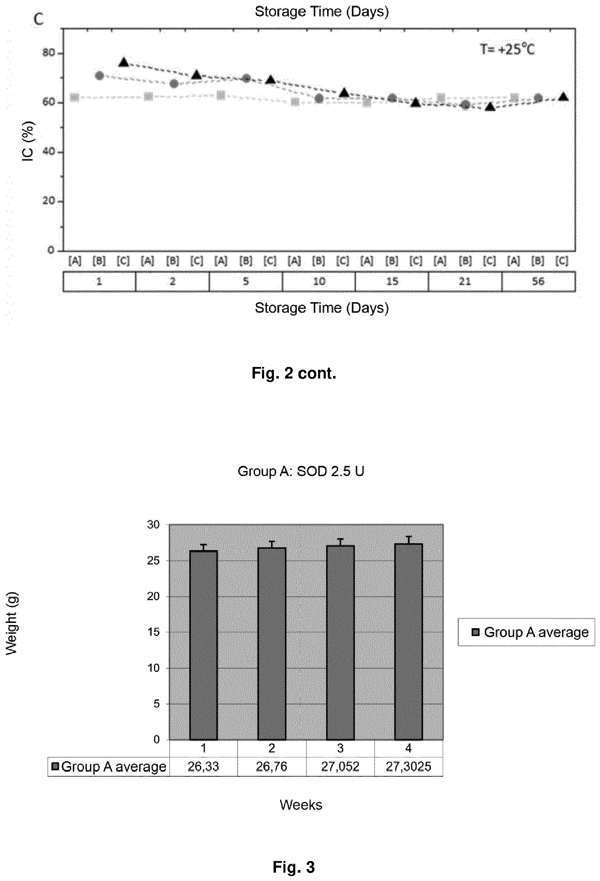

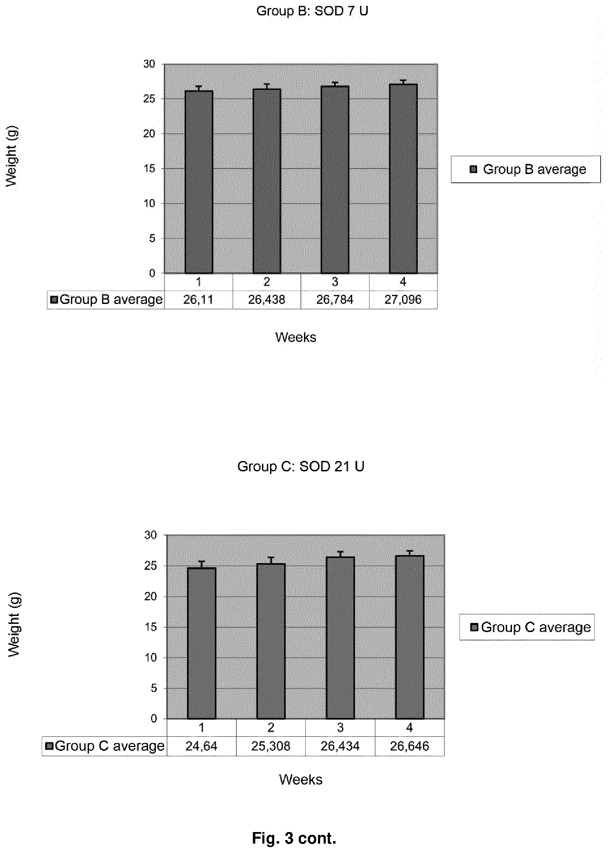

Figure 3 shows the graphical representation of the concentration of total antioxidant in the two main groups classified into subgroups according to the concentration of SOD that was tested, without differences between groups and subgroups, which supports the efficiency of the formula with SOD to maintain a total antioxidant activity within the values of normality, in human tears.

It is known that eyes with ocular surface dysfunction syndrome - dry eye - have an increased oxidative activity and a decreased antioxidant capacity. In this test it has been verified that with the two doses used of the SOD enzyme, the tear samples of the patients with dry eye syndrome matched their total antioxidant values to the values of the controls, as shown in Figure 3, suggesting that the contribution of the enzyme to the tear fluid induces the increase in the total antioxidant activity of the sample, which due to the disease was decreased and the pro-oxidant activity increased. That is, the administration of the composition of the invention containing SOD contributes to normalizing the values of antioxidant activity in tears of patients with dry eye disease and therefore bring them to the values of healthy subjects.

Example 5: Measurement of Inflammation Molecule Expression

This test was developed to assess the effectiveness of the formulation containing SOD in the eyes of healthy mice, evaluating the expression of inflammation molecules, specifically interleukin 6, which has been shown to play an essential role in the inflammation and immune response mechanisms of patients with ocular surface dysfunction syndrome - dry eye. Antioxidants have inherent anti-inflammatory properties, through the direct inhibition of EROX-mediated activation of NFkB signalling, which works as a transcription factor by promoting the expression of pro-inflammatory cytokines and chemokines.

Twenty mice (10 females and 10 males) have been studied and kept in cages and housing conditions, with 12 h/12 h light, humidity, temperature and feeding conditions (feed and water) controlled, and examined at baseline and at 15 days. Body weight is shown in Figure 7 We proceeded to instil in the right eye a drop of the SOD1 solution in 10 mice and a drop of the SOD2 solution in 10 mice. On the other hand, both groups were instilled a drop of the vehicle used in the left eye without the formulation.

The eye examination showed that the anterior segment structures were undamaged at the beginning of the study. The eyelids, conjunctiva, cornea, anterior chamber and its transparency, pupils and iris were examined. Blood was drawn from the submandibular vein of the animals, and determination of the proinflammatory molecule interleukin 6 (IL6) was performed by ELISA. Data were expressed as mean + SD in pg/mL, following protocol and manufacturer's instructions. The results obtained are shown in Figures 8 and 9.

The data from the measurement of IL6 in plasma of all experimental animals demonstrate that the two concentrations of the formula with SOD1 and SOD2 acted equally in terms of expression of the pro-inflammatory molecule. The two concentrations were equally effective in both females and males, which means that the formulation would be effective to act against the inflammatory mechanism that accompanies eye surface diseases, including ocular surface dysfunction (dry eye). In addition, the eye examination of the animals 15 days after the continued instillation of the product in its two concentrations showed that the eyelids, eyelashes, conjunctiva and cornea were not damaged by it. The animals also did not demonstrate behaviours consistent with discomfort or changes in their daily routine, so it follows that the product also did not interfere with their overall health.

**Claims**

1. A composition comprising:

   - superoxide dismutase (SOD) enzyme, in a concentration between 0.002 U/mL and 30,000 U/mL.
   - hyaluronic acid or any of its salts, in a concentration between 0.0001 and 10% weight/volume with respect to the total composition,
   - ethylenediaminetetraacetic acid (EDTA) or any of its salts, in a concentration between 0.0001 and 10% with respect to the total composition, and
   - a pH buffer selected from citrate, phosphate, ascorbate, borate.

2. The composition according to claim 1, wherein said SOD is selected from Cu/Zn-SOD, Mn-SOD, Ni-SOD and Fe-SOD.

3. The composition according to claim 2, wherein said SOD is Cu/Zn-SOD.

4. The composition according to any of claims 1 to 3, wherein said SOD can be of plant, animal, human or synthetic origin.

5. The composition according to claim 4, wherein said SOD is of animal origin.

6. The composition according to any of claims 1 to 5, wherein the SOD is in a concentration between 0.2 and 20,000 U/mL.

7. The composition according to claim 6, wherein the SOD is in a concentration between 1 and 500 U/mL.

8. The composition according to claim 7, wherein the SOD is in a concentration between 10 and 250 U/mL.

9. The composition according to any of claims 1 to 8, wherein hyaluronic acid is in a concentration between 0.05 and 5% weight/volume with respect to the total composition.

10. The composition according to claim 9, wherein hyaluronic acid is in a concentration of 0.1% weight/volume with respect to the total composition.

11. The composition according to any of claims 1 to 10, wherein the EDTA, is in a concentration comprised between 0.05 and 5% weight/volume with respect to the total composition.

12. The composition according to claim 11, wherein the EDTA is in a concentration of 0.1% weight/volume.

13. The composition according to any of claims 1 to 12, wherein the EDTA is in the form of sodium salt.

**14.** The composition according to any of claims 1 to 13 wherein the buffer is sodium borate.

**15.** The composition according to any of claims 1 to 14, further comprising a salt selected from NaCl, KCl, $MgCl_2$, $CaCl_2$.

**16.** The composition according to claim 15, wherein the salt is NaCl.

**17.** The composition according to any of claims 1 to 16, **characterized by** having a pH between 7 and 7.5.

**18.** The composition according to claim 17, **characterized by** having a pH of 7.3.

**19.** Pharmaceutical composition comprising the composition according to any of claims 1 to 18 and at least one pharmaceutically acceptable carrier.

**20.** A pharmaceutical composition according to claim 19, wherein said pharmaceutical composition is formulated in the form of eye drops, artificial tear solution, solution for injection, spray, gel, ointment or cream.

**21.** Composition according to claims 1 to 18 for use as a medicinal product.

**22.** Composition according to claims 1 to 18 for use for the treatment and/or prevention of eye diseases.

**23.** A composition for use according to claim 22, wherein the eye disease is selected among the following: dry eye syndrome, conjunctivitis with inflammatory component, chronic blepharitis, non-infectious keratitis, keratoconus, non-infectious uveitis and/or glaucoma.

**24.** Composition according to claims 1 to 18 for use in the treatment and/or prevention of the inflammatory response and oxidative stress caused postoperatively by eye surgery.

**25.** Composition for use according to claim 24, wherein the post-operative period is in surgery of the anterior segment of the eye and/or laser treatment.

HOOH ——————————→ HO* hydroxyl radical

Fe²⁺  Fe³⁺      HO⁻
Cu²⁺  Cu¹⁺
Cr⁵⁺  Cr⁶⁺
Ni²⁺  Ni³⁺

Superoxide radical anion  O₂·⁻

O₂

**Fig. 1**

A

T= -21°C

**Fig. 2**

Fig. 2 cont.

Fig. 2 cont.

**Fig. 2 cont.**

Group A: SOD 2.5 U

| ▣ Group A average | 26,33 | 26,76 | 27,052 | 27,3025 |

Weeks

**Fig. 3**

Group B: SOD 7 U

| ▣Group B average | 1 | 2 | 3 | 4 |
|---|---|---|---|---|
| | 26,11 | 26,438 | 26,784 | 27,096 |

Weeks

Group C: SOD 21 U

| ▣Group C average | 1 | 2 | 3 | 4 |
|---|---|---|---|---|
| | 24,64 | 25,308 | 26,434 | 26,646 |

Weeks

**Fig. 3 cont.**

**Fig. 4**

Group A: SOD 2,5 U

**Fig. 5**

Group B: SOD 7 U

Weeks

Group C: SOD 21 U

Weeks

**Fig. 5 cont.**

## Total Antioxidant Activity (mM) in Tear

| | |
|---|---|
| ☐ SOD1 controls | 0,42059808 |
| ■ SOD2 controls | 0,42060192 |
| ▨ SOD1 dry eye | 0,42041472 |
| ▦ SOD2 dry eye | 0,42036864 |

**Fig. 6**

## Mice weight (g)

| | |
|---|---|
| Female weight | 19,683 |
| Male weight | 27,705 |

**Fig. 7**

IL6 Expression in mouse baseline plasma

| | |
|---|---|
| ☐ SOD1 Females | 0,176469476 |
| ■ SOD2 Females | 0,176214864 |
| ▨ SOD1 Males | 0,176165468 |
| ▨ SOD2 Males | 0,176157412 |

**Fig. 8**

IL6 expression in mouse plasma at
15 days of treatment

| | |
|---|---|
| ☐ SOD1 Females | 0,176186456 |
| ■ SOD2 Females | 0,176138968 |
| ▨ SOD1 Males | 0,176138544 |
| ▨ SOD2 Males | 0,1762049 |

**Fig. 9**

## INTERNATIONAL SEARCH REPORT

| International application No. |
| --- |
| PCT/ES2022/070486 |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)
A61K, A61P

Documentation searched other than minimum documentation to the extent that such documents are included in the fields searched

Electronic data base consulted during the international search (name of data base and, where practicable, search terms used)

EPODOC, INVENES, WPI, MEDLINE, NPL, EMBASE, BIOSIS, Internet

**C. DOCUMENTS CONSIDERED TO BE RELEVANT**

| Category* | Citation of document, with indication, where appropriate, of the relevant passages | Relevant to claim No. |
| --- | --- | --- |
| P, X | CN 114177280 A (SHANGHAI WEICON OPTICS CO LTD) 15/03/2022, the whole document | 1-25 |
| Y | TW 201722412 A (ROHTO PHARMA) 01/07/2017, the whole document | 1-25 |
| Y | US 2003228299 A1 (DROY-LEFAIX ET AL) 11/12/2003, the whole document | 1-25 |
| A | EP 2433640 A1 (VISIOTACT PHARMA) 28/03/2012, the whole document | 1-25 |
| A | CN 102697713 A (ZHEJIANG JIANFENG PHARM CO LTD) 03/10/2012, the whole document | 1-25 |

☒ Further documents are listed in the continuation of Box C.  ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
| --- | --- |
| 06/10/2022 | (07/10/2022) |

| Name and mailing address of the ISA/ <br><br> OFICINA ESPAÑOLA DE PATENTES Y MARCAS <br> Paseo de la Castellana, 75 - 28071 Madrid (España) <br> Facsimile No.: 91 349 53 04 | Authorized officer <br> M. Cumbreño Galindo <br><br><br> Telephone No. 91 3496880 |
| --- | --- |

Form PCT/ISA/210 (second sheet) (January 2015)

**EP 4 374 871 A1**

## INTERNATIONAL SEARCH REPORT

International application No.

PCT/ES2022/070486

C (continuation).                    DOCUMENTS CONSIDERED TO BE RELEVANT

| Category * | Citation of documents, with indication, where appropriate, of the relevant passages | Relevant to claim No. |
|---|---|---|
| A | Colirio. Wikipedia, 01/06/2021 [on line][retrieved the 29/11/2021]. Retrieved from <URL: https://es.wikipedia.org/w/index.php?title=Colirio&oldid=135996482#Composici%C3%B3n>, the whole document | 1-25 |
| A | CN 112336660 A (SICHUAN FUYI ZHANGZHE CULTURE COMMUNICATION CO LTD) 09/02/2021, the whole document | 1-25 |

Form PCT/ISA/210 (continuation of second sheet) (January 2015)

## INTERNATIONAL SEARCH REPORT
Information on patent family members

International application No.

PCT/ES2022/070486

| Patent document cited in the search report | Publication date | Patent family member(s) | Publication date |
|---|---|---|---|
| CN114177280 A | 15.03.2022 | NONE | |
| TW201722412 A | 01.07.2017 | JPWO2017094508 A1 | 23.08.2018 |
| | | JP6592527B B2 | 16.10.2019 |
| | | TWI743066B B | 21.10.2021 |
| | | WO2017094508 A1 | 08.06.2017 |
| US2003228299 A1 | 11.12.2003 | JP2004537522 A | 16.12.2004 |
| | | WO02098345 A1 | 12.12.2002 |
| | | WO02098345 A8 | 09.01.2003 |
| | | FR2832637 A1 | 30.05.2003 |
| | | FR2832637 B1 | 30.07.2004 |
| | | FR2832636 A1 | 30.05.2003 |
| | | EP1392352 A1 | 03.03.2004 |
| | | CN1512893 A | 14.07.2004 |
| | | CA2449825 A1 | 12.12.2002 |
| | | BR0209523 A | 13.07.2004 |
| EP2433640 A1 | 28.03.2012 | ES2774659T T3 | 22.07.2020 |
| | | PL2433640T T3 | 01.06.2020 |
| | | PT2433640T T | 03.04.2020 |
| | | US2013195985 A1 | 01.08.2013 |
| | | CA2812999 A1 | 29.03.2012 |
| | | EP2618832 A2 | 31.07.2013 |
| | | WO2012038512 A2 | 29.03.2012 |
| | | WO2012038512 A3 | 18.05.2012 |
| CN102697713 A | 03.10.2012 | CN102697713B B | 07.08.2013 |
| CN112336660 A | 09.02.2021 | NONE | |

Form PCT/ISA/210 (patent family annex) (January 2015)

21

**INTERNATIONAL SEARCH REPORT**

International application No.

PCT/ES2022/070486

CLASSIFICATION OF SUBJECT MATTER

*A61K38/44* (2006.01)
*A61K31/728* (2006.01)
*A61K31/198* (2006.01)
*A61P27/02* (2006.01)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030228299 A
- WO 03082081 A
- US 20050032914 A
- US 20100159029 A
- WO 2009129859 A
- CN 101243875
- US 20130195985 A

### Non-patent literature cited in the description

- **S. DI MEO ; T. REED ; P. VENDITTI ; V. M. VICTOR.** Role of ROS and RNS Sources in Physiological and Pathological Conditions. *Oxid. Med. Cell Longer,* 2016, vol. 2016, 1245049
- **I. VOULDOUKIS ; D. LACAN ; C. KAMATE ; P. COSTE ; A. CALENDA ; D. MAZIER ; M. CONTI ; B. DUGAS.** *Journal of Ethnopharmacology,* 2004, vol. 94 (1), 67-75
- **CHOI W ; LIAN C ; YING L, et al.** Expression of lipid peroxidation markers in the tear film and ocular surface of patients with non-Sjogren syndrome: potential biomarkers for dry eye disease. *Curr Eye Res.,* 2016, vol. 41, 1143-1149
- **SEEN S ; TONG L.** Dry eye disease and oxidative stress. *Acta Ophthalmologica,* 2017, vol. 96 (4), e412-e420
- **CHOY CK ; BENZIE IF ; CHO P.** Ascorbic acid concentration and total antioxidant activity of human tear fluid measured using the FRASC assay. *Invest Ophthalmol Vis Sci.,* 2000, vol. 41, 3293-8